# EUROPÄISCHE PATENTANMELDUNG

(11) **EP 0 552 854 A1**
(43) Veröffentlichungstag der Anmeldung: **28.07.1993**
(21) Anmeldenummer: 93250019.2
(22) Anmeldetag: 19.01.1993
(51) Int. Cl.: A61M 5/168

(54) **Dosiervorrichtung für übertragungsgeräte der Infusions- und Transfusionstechnik**

(30) Priorität: 21.01.1992 DE 4201416
(71) Anmelder: Mommer, Andreas, D-52064 Aachen (DE)
(72) Erfinder: Mommer, Andreas, D-52064 Aachen (DE)

(57) **Zusammenfassung**

Die Dosiervorrichtung der Infusions- und Transfusionstechnik zur Regulierung des Durchflußquerschnittes in einer Flüssigkeitsleitung für Flüssigkeitsinfusionen- und transfusionen, besteht aus einem Gehäuse (1) mit je einer in dessen Ventilraum (2) in unterschiedlichen Ebenen mündende Einlauf- (9) und Auslauföffnung (10) und einem in dem Ventilraum angeordneten, im wesentlichen zylindrischen, verdrehbaren Küken (3) mit einem Verstellorgan, welches eine ununterbrochene Nut aufweist, die sich über mindestens zwei, in ihrer Höhe der Lage der Einlauf- und Auslauföffnung entsprechenden, Ebenen erstreckt, von der ein Teil als umlaufende Ringnut (4) mit gleichbleibendem Querschnitt, ein zweiter Teil als Dosiernut (5) mit auf ihrer Länge sich kontinuierlich veränderndem Querschnitt und ein drittes Teilstück, als Verbindungsnut (6) zwischen den beiden anderen Abschnitten der Nut, mit konstantem oder veränderlichem Querschnitt ausgebildet ist. Dabei bestimmt die Position des als Dosiernut (5) ausgebildeten Nutabschnitts vor der Einlauf- oder Auflauföffnung den wirksamen Durchflussquerschnitt und damit die Tropfrate.

## Beschreibung

Die Erfindung betrifft eine Dosiervorrichtung für Übertragungsgeräte der Infusions- und Transfusionstechnik
Bei der Durchführung von Infusionen oder Transfusionen wird der Stopfen eines die Lösung enthaltenden Behälters mit dem Dorn eines Übertragungsgerätes durchstochen und er Behälter mit nach unten weisender Öffnung aufgehängt, so daß die Flüssigkeit in Form von Tropfen ausläuft. Die Flüssigkeit wird zunächst einem Zwischenbehälter zugeführt und gelangt von dort über einen flexiblen Schlauch zum Patienten. Das Auslaufen der Flüssigkeit aus dem Zwischenbehälter, und damit die Zufuhr zum Patienten, wird durch eine Klemmvorrichtung reguliert, die den Schlauch abquetscht und damit den Durchflußquerschnitt verringert. Die Klemmvorrichtung weist eine Rolle auf, die in bezug auf einen Keil verstellbar ist, und zwischen Rolle und Keil wird der Schlauch eingeklemmt.
Die Tropfenzahl pro Zeiteinheit läßt sich mit dieser Vorrichtung nicht über einen längeren Zeitraum konstant halten, da sich der flexible Schlauch infolge seiner Elastizität verformen kann und mit der Zeit eine gewisse Quellung des Materials auftritt.
Hinzu kommen Ermüdungen des Materials, so daß die Elastizität nachläßt und sich nach längerem Gebrauch die Innenseite des Schlauches verengt und der Durchlaß sich ändert. Soll die dem Patienten zuzuführende Flüssigkeitsmenge erhöht werden, so kann es vorkommen, daß der Schlauch sich nach Lösen der Klemme nicht sofort in seinen Ursprungszustand zurückbildet, so daß dem Patienten nicht die benötigte Flüssigkeitsmenge mit der erforderlichen Schnelligkeit zugeführt werden kann. Weiterhin ist eine ständige Nachregulierung der Klemmvorrichtung notwendig, um eine konstante Tropfgeschwindigkeit zu erreichen.

Des weiteren sind Dosiervorrichtungen (DE-PS 27 35 955, DE GM 77 22 466, US-PS 29 11 008) bekannt, die aus einem Ventilgehäuse und einem Küken bestehen, wobei das Küken zum Beispiel eine umlaufende Nut mit sich kontinuierlich verändernder Querschnittsfläche aufweist. Die über den Einlaufstutzen in das Ventilgehäuse eintretende Flüssigkeit gelangt dabei in die umlaufende Nut, deren kleinster sich zwischen Einlauf- und Auslaufstutzen befindlicher Teil die Tropfrate bestimmt.

Durch Verdrehen des Kükens im Ventilgehäuse um seine Längsachse kann dieser Querschnitt zum Beispiel kontinuierlich verändert werden. Der Nachteil dieser Dosiervorrichtungen ist zum Beispiel (DE PS 27 35 955) die konstruktionsbedingte Begrenzung des Dosierweges auf maximal 180 Grad Drehung, so daß eine feinabgestimmte Dosierung, insbesondere im Bereich kleiner bis mitlerer Durchflußraten, nur begrenzt möglich ist, oder (US-PS 2 911 008) der hohe formtechnische Aufwand bei der Massenfertigung.

Die Aufgabe der Erfindung ist es, eine Dosiervorrichtung zu schaffen, die bei preiswerter Herstellbarkeit und hohen Anforderungen an die Funktionssicherheit sowie der Genauigkeit und Zuverlässigkeit einen möglichst großen Dosierweg aufweist, der ein genaues und feinühliges Dosieren im Bereich geringer Tropfraten bzw. Durchflußmengen ermöglicht.

Zur Lösung dieser Aufgabe ist erfindungsgemäß vorgesehen, daß ein Gehäuse mit zwei lotrecht zueinanderstehenden, aber in verschiedenen Ebenen abstehenden Anschlußstutzen einen senkrecht zu den Anschlustutzen verlaufenden im wesentlichen zylindrischen Ventilraum aufweist, in den die Anschlußstutzen in verschiedenen Ebenen münden, und daß in dem Ventilraum ein drehbares Küken angeordnet ist, welches eine ununterbrochene Nut aufweist, deren erster Teil in der Ebene der Einlauföffnung und deren letzter Teil in der Ebene der Auslauföffnung liegt und die über einen senkrecht zu ihnen verlaufenden Abschnitt miteinander verbunden sind, und die durch Drehung des Kükens um seine Längsachse unterschiedliche Durchlaßquerschnitte vor der Auslauföffnung freigibt, während der Querschnitt des ersten Teils der Nut vor der Einlauföffnung konstant bleibt.

In vorteilhafter Ausgestaltung der Erfindung weist die ununterbrochene Nut auf der Mantelfläche des Kükens in ihrem unteren Teil eine als Ringnut mit konstantem Querschnitt ausgebildeten Abschnitt in Höhe der Einlauföffnung auf dem vollen Umfang und eine als Dosiernut mit kontinuierlich sich veränderndem Querschnitt ausgebildeten Abschnitt in Höhe der Auslauföffnung, auf. Beide Abschnitte sind durch eine Verbindungsnut, die vorzugsweise senkrecht zu den beiden übrigen Teilstücken der Nut verläuft und einen konstanten Durchlaßquerschnitt aufweist, verbunden. Die Flüssigkeit, welche von der Tropfenkammer Über die Einlauföffnung in die Dosiervorrichtung einläuft, gelangt in den umlaufenden Ringnutabschnitt des Kükens, dessen Aussenfläche im übrigen abdichtend an der Wandung des Ventilraumes anliegt, und über den Verbindungsnutabschnitt in den Dosiernutabschnitt und von dort aus über die Auslauföffnung und einen flexiblen Schlauch zum Patienten.

Der Querschnitt des Dosiernutabschnittes, der sich vor der Mündung der Auslauföffnung befindet bestimmt den Durchflusswiderstand und damit die Tropfrate. Durch Drehen des Kükens um seine Längsachse kann der wirksame Dosiernutquerschnitt verändert werden. Die Querschnittsveränderung kann beispielsweise durch eine Änderung der Nutbreite oder vorzugsweise eine Änderung der Nuttiefe in Umfangsrichtung erfolgen. Auch kann entsprechend die Querschnittform des Dosiernutabschnitts variieren. Der Querschnitt kann beispielsweise rechteckig, halbkreis-, oder keilförmig sein.

Wichtig ist, daß durch Drehen des Kükens derjenige Bereich des Dosiernutabschnitts, der sich vor der Mündung der Auslauföffnung befindet, in seinem Querschnitt verändert wird. Die Querschnittform des Ringnutabschnitts ist auf seiner vollen Länge konstant und gleichbleibend in ihrer Größe.

Es sei darauf hingewiesen, daß eine sinngemäße Umkehrung der Lage der Nutabschnitte und damit verbundene Verlagerung der Drosselung des Durchlasses von der Auslauföffnung zur Einlauföffnung denkbar ist und im Rahmen der Erfindung liegt.

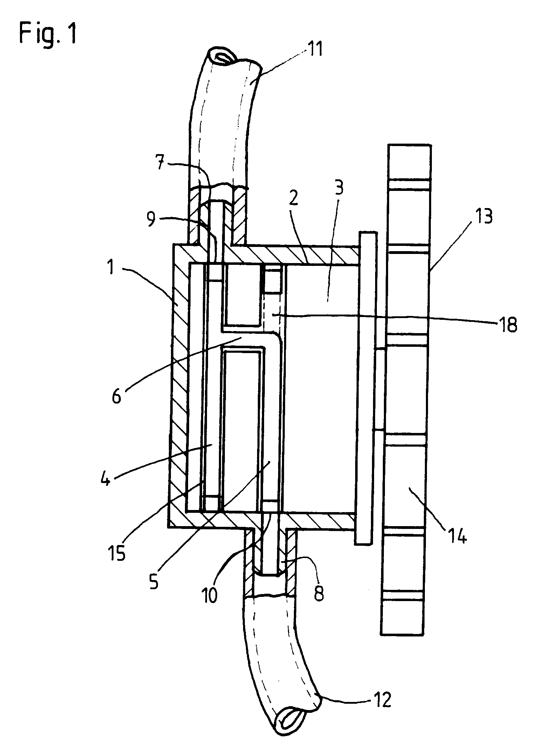

Im folgendem werden unter Bezugnahme auf die Figuren einige Ausführungsbeispiele der Erfindung näher erläutert.
Figur 1 zeigt einen Teilschnitt durch eine erste Ausführungsform der Dosiervorrichtung in Längsrichtung,
Figur 2 zeigt eine Seitenansicht des Kükens der Dosiervorrichtung nach Figur 1,
Figur 3 zeigt eine Draufsicht des Kükens nach Figur 1 oder 2 in einem Teilschnitt,
Figur 4 zeigt eine Detailansicht des Ausschnittes "X" des Kükens von Figur 2,
Figur 5 zeigt eine zweites Ausführungsbeispiel des Ringnutabschnitts gemäß Anspruch 6,
Figur 6 zeigt eine Draufsicht des Handrades nach Figur 1,
Figur 7 zeigt eine weiteres Ausführungsbeispiel des Ventilgehäuses nach Figur 1 gemäß Anspruch 5,
Die Dosiervorrichtung nach Figur 1 besteht aus einem einseitig geschlossenem, im wesentlichen zylindrischen Ventilgehäuse 1, in dessen Ventilraum 2 ein abdichtend passendes zylindriches Küken 3 angeordnet ist, auf dessen an der Wandung des Ventilraumes 2 anliegenden Aussenfläche eine ununterbrochene Nut, bestehend aus einem auf dem vollen Umfang des Kükens 3 ausgebildetem Ringnutabschnitt 4, einem sich über den grössten Teil des Umfangs erstreckenden Dosiernutabschnitt 5 und einem Verbindungsnutabschnitt 6 zwischen dem Ringnutabschnitt 4 und dem Dosiernutabschnitt 5, ausgebildet ist.

Der Ventilraum 2 des Ventilgehäuses 1 steht mit einer Einlauföffnung 9 und einer Auslauföffnung 10 in Verbindung. Die Einlauföffnung 9 ist dabei in gleicher Höhe wie der Ringnutabschnitt 4 und die Auslauföffnung 10 in gleicher Höhe wie der Dosiernutabschnitt 5 im Ventilgehäuse 1 angebracht. An der Aussenseite des Ventilgehäuses 1 sind die sich lotrecht gegenüberliegende Einlauföffnung 9 und Auslauföffnung 10 durch ein Rohrstück 7,8 über die äusseren Konturen des Ventilgehäuses 1 hinaus verlängert. Auf diesen Rohrstücken 7,8 steckt ein flexibler Schlauch 11,12. Der einlaufseitige, obere Schlauch 11 kommt von einem gebräuchlichen Übertragungsgerät, z.B. einer Tropfenkammer, und der untere, auslaufseitige Schlauch 12 führt zum Patienten.

Bei Drehung des Kükens 3 wird der Ringnutabschnitt 4 mit konstant bleibendem Querschnitt an der Einlauföffnung 9 vorbeigedreht, wobei die Öffnung in keiner Stellung des Kükens 3 verdeckt wird, und die einströmende Flüssigkeit verteilt sich auf dem gesammten Umfang des Ringnutabschnittes 4, gelangt dann zum Verbindungsnutabschnitt 6 und über diesen zum Dosiernutabschnitt 5, welcher im bezug auf die Auslauföffnung 10 bei Drehung des Kükens 3 seinen Querschnitt ändert, so daß sich eine Änderung des Querschnittes vom vollständigen Verschluß bishin zur maximalen Öffnung und umgekehrt ergibt.

Die maximale Öffnung ist erreicht, wenn sich die Stelle des Dosiernutabschnitts 5, an der der Übergang in den Verbindungsnutabschnitt 6 ausgebildet ist, vor der Auslauföffnung 10 befindet. Der Flüssigkeitsdurchfluss ist abgeriegelt, wenn sich der ca. 5 - 20 Grad breite Bereich 18 in der Höhe des Dosiernutabschnittes 5 des Kükens 3, der abdichtend an der Wandung des Ventilraumes 2 anliegt, vor der Auslauföffnung 10 befindet.

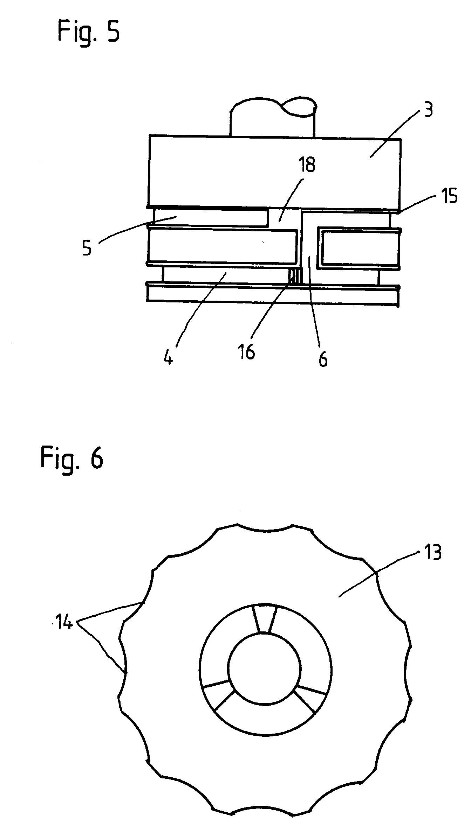

Die Drehung des Kükens 3 erfolgt über ein Handrad 13, welches mit seinem einem Ende einstückig am Küken 3 ausgebildet sein kann. Zur feinfühligen Handhabung können auf dem gesamten Umfang des Handrades 13, wie in Figur 6 dargestellt, fingergerecht geformte Griffmulden 14 ausgebildet werden. Diese Griffmulden 14 ermöglichen eine feinfühlige und exakte Einstellung des Kükens 3 über das Handrad 13 mit zwei Fingern. Die Drehachse des Handrades 13 ist senkrecht zur Längsachse der Schlauchstücke 11,12 und der Rohrstücke 7,8.

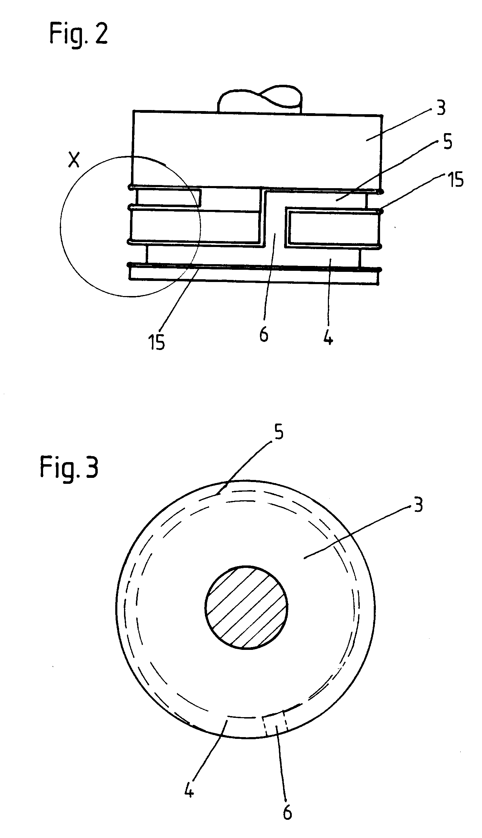

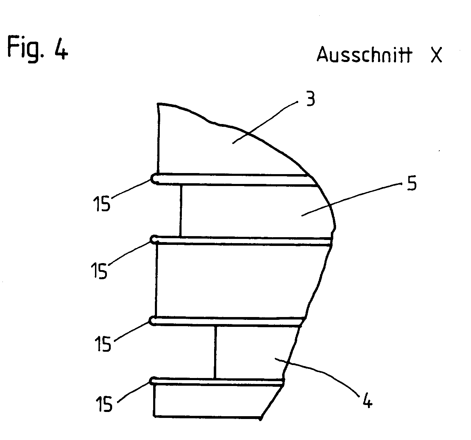

Figur 2 und 3 zeigen die vorzugsweise Anordnung der Nutabschnitte 4, 5 und 6 in zwei Ebenen in der Mantelfläche des Kükens 3. Diese können, wie das in Figur 4 gezeigte Ausführungsbeispiel darstellt, in einer vorteilhaften Weiterentwicklung an den Kanten der Nutabschnitte 4,5 und 6 angeformte, umlaufende Dichtwülste 15 aufweisen, die eine sichere Abdichtung der gegenüber dem übrigen Ventilraum 2, bei gleichzeitiger Reduzierung des aufzuwendenden Drehmomentes bei der Verdrehung des Kükens 3, bewirken, da bei diesem Ausführungsbeispiel nicht die gesamte Mantelfläche des Kükens 3 an der Wandung des Ventilraumes 2 abdichtend anliegen muß.

Die in Figur 5 gezeigte weitere Ausführungsmöglichkeit des Ringnutabschnittes 4 gemäß Anspruch 6 gewährleistet eine sichere Entlüftung der Nutabschnitte 4, 5 und 6 bei der erstmaligen Benutzung der Dosiervorrichtung.

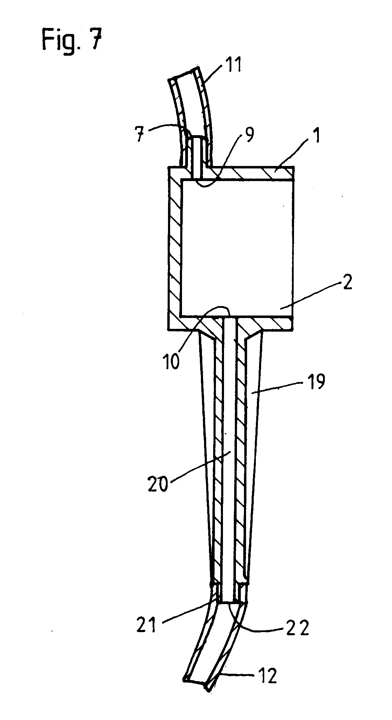

Das in Figur 7 gemäß Anspruch 5 dargestellte Ausführungsbeispiel für das Gehäuse 1 mit einstückig angeformten Griffstück 19 und Rohrstutzen 21 und auf den Rohrstutzen 21 aufgestecktem Schlauchstück 12 weist eine durch die Längsachse des Griffstückes 19 verlaufende durchgehende Bohrung 20 mit der Mündung 10 im Ventilraum 2 und der Mündung 22 im Rohrstutzen 21, auf. Dieses Ausführungsbeispiel ermöglicht gegenüber einer zweiteiligen, zusammensteckbaren Ausführung neben einer erheblichen Reduzierung der Werkzeug- und damit Herstellungskosten für das Gehäuse 1 auch eine stabilere, unlösbare Verbindung der beiden Teilstücke.

Die erfindungsgemäße Dosiervorrichtung ermöglicht eine feinfühlige und exakte Dosierung. Durch die horizontale Versetzung der Nutabschnitte ist es möglich, den Dosierweg über mehr als 180 Grad Drehung des Kükens auszudehnen, da es durch die Anordnung in zwei Ebenen nur einen Bereich von ca. 5 - 20 Grad gibt, der abdichtend an der Wandung des Ventilgehäuses anliegt und der, je nach Aufteilung der Nutabschnitte, die Einlauf- oder Auslauföffnung verdeckt.

Insbesondere die Einregulierung kleiner bis mittlerer Durchflussmengen bis ca. 100 ml/h wird durch die Ausdehnung des Dosiernutabschnittes über ca. 340 Grad vereinfacht und zum Teil erst ermöglicht, wodurch die erfindungsgemäße Dosiervorrichtung eine wichtige Forderung der Anwender erfüllt.

Die Teile der Dosiervorrichtung sind als Massenartikel aus dafür geeigneten Kunstoffen preiswert herzustellen.

## Patentansprüche

1. Dosiervorrichtung für Übertragungsgräte der Infusions- und Transfusionstechnik bestehend aus einem Ventilgehäuse (1) mit in dem Ventilgehäuse (1) angeordneter Einlauföffnung (9) und Auslauföffnung (10) und mit diesen in Verbindung stehenden nach aussen einstückig angeformten und in Bezug auf den Boden des Ventilgehäuses, horizontal versetzt aber im übrigen vorzugsweise lotrecht zueinander angeordneten Rohrstücken (7,8), und einem in dem Ventilgehäuse angeordneten, im wesentlichen zylindrischen Küken (3), **dadurch gekennzeichnet,** daß das Küken (3) eine, auf seiner Mantelfläche ausgebildete und in mindestens drei Abschnitte, einen Ringnutabschnitt (4), einen Dosiernutabschnitt (5) und einen Verbindungsnutabschnitt (6), unterteilte, Nut aufweist, deren Abschnitte je eine von den anderen Nutabschnitten getrennte Funktion ausüben. Und von denen der erste Abschnitt (4) und der letzte Abschnitt (5) mit der Einlauföffnung (9) und der Auslauföffnung (10) in Verbindung stehen.

2. Dossiervorrichtung nach Anspruch 1, **dadurch gekennzeichnet**, daß der, mit auf dem gesamten Umfang konstantem Querschnitt in der Mantelfläche des Kükens (3) ausgebildete, Ringnutabschnitt (4), unabhängig von der Drehstellung des Kükens immer mit der sich in der entsprechenden Ebene befindenden Einlauföffnung (9) in Verbindung steht und über die gesamte Drehung des Kükens (3) den konstanten Ein- oder Auslauf der Flüssigkeit ermöglicht.

3. Dosiervorrichtung nach Anspruch 1, **dadurch gekennzeichnet**, daß der, auf der gesamten Länge mit konstantem Querschnitt ausgestaltete Nutabschnitt (6), ohne die Durchflußmenge durch die Dosiervorrichtung zu beeinflussen, die Verbindung zwischen den anderen Nutabschnitten (4 und 5) darstellt und vorzugsweise senkrecht zu den beiden anderen Abschnitten in der Mantelfläche des Kükens (3) ausgestaltet ist.

4. Dosiervorrichtung nach Anspruch 1, **dadurch gekennzeichnet**, daß der Nutabschnitt (5) mit auf der gesamten Länge sich kontinuierlich änderndem Querschnitt durch Drehung des Kükens (3) im Gehäuse (1) an der, sich in der gleichen Ebenen befindenden, Auslauföffnung (10) vorbeigedreht wird und alleine den aktiven Durchflußquerschnitt vor der Auslauföffnung (10), von der vollständigen Abriegelung des Durchflußes bis zum ungehinderten Durchfluß und umgekehrt, auf einem Drehweg von ca. 340 bis 355 Grad, reguliert.

5. Dosiervorrichtung nach Anspruch 1, **dadurch gekennzeichnet,** daß das Gehäuse (1) mit einem einstückig angeformten Griffstück (19) ausgebildet wird, welches eine dem Mündungsdurchmesser der Auslauföffnung (10) entsprechende, durchgehende Bohrung (20) aufweist und an dem das Schlauchteil (12) an einem dafür einstückig angeformten Rohrstutzen (21) aufgesteckt wird.

6. Dosiervorrichtung nach Anspruch 1 und 4, **dadurch gekennzeichnet,** daß in dem Ringnutabschnitt (4) an der Mündung des Verbindungsnutabschnittes (6) eine Flüssigkeitssperre (16) ausgestalltet werden kann, deren Abstand zur Wandung des Ventilraumes (2) und Formgebung so gewählt sind, daß zum einen Luft, die sich im Ringnutabschnitt (4) befindet, die Sperre (16) passieren kann, Flüssigkeit aber aufgehalten wird und zum anderen die durch die Einlauföffung (9) einströmende Flüssigkeit nicht behindert wird.

7. Dosiervorrichtung nach Anspruch 1 bis 4 , **dadurch gekennzeichnet,** daß das Handrad (13) ergonomisch ausgeformte Griffmulden (14) aufweist.

8. Dosiervorrichtung nach Anspruch 1 bis 7, **dadurch gekennzeichnet,** daß die Mantelfläche des Kükens (3) an den, die Nutabschnitte (4,5,6) begrenzenden Kanten, ununterbrochene Dichtwülste (15) aufweist.
